# EUROPEAN PATENT APPLICATION

(11) **EP 2 570 190 A1**
(43) Date of publication of application: **20.03.2013**
(21) Application number: 11007522.3
(22) Date of filing: 15.09.2011
(51) Int. Cl.: B05B 1/34

(54) **Spray nozzle for dispensing a fluid and sprayer comprising such a spray nozzle**

(71) Applicant: Braun GmbH, 61476 Kronberg/Taunus (DE)
(72) Inventor: Bó, Paolo Dal, 64646 Heppenheim (DE)

(57) **Abstract**

The present invention relates to a spray nozzle (2) for dispensing a fluid comprising a fluid chamber (40) for receiving the fluid, at least one feeding channel (42) for feeding the fluid from the fluid chamber (40) radially inward into a swirl chamber (44) and an outlet channel (18) with an entrance end (54) facing the swirl chamber (44) and an exit end (56) for discharging the fluid to the environment (58) of the spray nozzle (2). The outlet channel (18) tapers in the flow direction of the fluid. The present invention further relates to a sprayer comprising such a spray nozzle (2).

## Description

### FIELD OF THE INVENTION

The present invention relates to a spray nozzle for dispensing a fluid comprising a fluid chamber, at least one feeding channel for feeding the fluid from the fluid chamber radially inward into a swirl chamber and an outlet channel with an entrance end facing the swirl chamber and an exit end for discharging the fluid to the environment of the spray nozzle. The present invention further relates to sprayers comprising such a spray nozzle.

### BACKGROUND OF THE INVENTION

Spray nozzles for dispensing fluids are well known in the state of the art. Usually, the known spray nozzles comprise a fluid chamber, at least one feeding channel for feeding the fluid from the fluid chamber radially inward into a swirl chamber and an outlet channel with an entrance end facing the swirl chamber and an exit end for discharging the fluid to the environment of the spray nozzle. The cross-section of the outlet channel has a constant form and a constant cross-sectional area in the flow direction of the fluid.

The known spray nozzles for dispensing fluids have proved themselves. However, a relatively high pump pressure is necessary, especially if highly viscous or tough-flowing fluids have to be dispensed. Above this, it is desirable to reduce the decrease of pressure within the known spray nozzles. Further, the known spray nozzles do not allow a flexible adjustment of the spray pattern, the volume flow rate and so forth. Furthermore, the production and prototyping of the known spray nozzles may be complicated.

It is therefore an object of the present invention to provide a spray nozzle for dispensing a fluid, the spray nozzle reducing the necessary pump pressure and the decrease of pressure within the nozzle. In other words, it is an object of the present invention to provide a spray nozzle generating the same particle size as conventional nozzles at a lower pressure or generating a spray with a smaller particle size at the same pressure. It is a further object of the present invention to provide a sprayer with a spray nozzle according to the invention with reduced pump pressure and a reduced decrease of pressure within the spray nozzle. In other words, it is an object of the present invention to provide a spray with the same particle size at a lower pressure, or to generate a spray with smaller particle size at the same pressure.

### SUMMARY OF THE INVENTION

The above-mentioned problem is solved by a spray nozzle and a sprayer as described in claims 1 and 15, respectively. Preferred and advantageous embodiments of the invention are described in the subclaims.

The present invention is directed to a spray nozzle for dispensing a fluid. The spray nozzle comprises a fluid chamber for receiving the fluid. It is preferred, if the fluid chamber is configured as a ring chamber, which is more preferably in fluid connection with a fluid storage chamber for storing the fluid. Said ring chamber is most preferably connecting with the swirl chamber, which will be described later. The spray nozzle further comprises at least one feeding channel for feeding the fluid from the fluid chamber radially inward into a swirl chamber. Usually, one feeding channel may be sufficient, however, it has been found out that two, preferably three, or more feeding channels effect a better production of swirl within the swirl chamber and consequently a more symmetrical spray pattern. It is further preferred, if the feeding channel tangentially leads into the swirl chamber. According to the invention, there is further provided an outlet channel. The outlet channel has an entrance end facing the swirl chamber and an exit end for discharging the fluid to the environment of the spray nozzle. In order to provide a spray nozzle needing a relatively low pump pressure and which is applicable even if highly viscous or tough-flowing fluids have to be dispensed, the outlet channel tapers in the flow direction of the fluid. It has been found out that the decrease of pressure within the spray nozzle could be reduced by using the tapered outlet channel. Further, the outlet channel tapering in the flow direction has positive effects on the spray pattern.

Basically, the outlet channel may taper stepwise in the flow direction. However, in a preferred embodiment of the spray nozzle according to the invention the outlet channel tapers steadily, thereby achieving a further reduction of the necessary pump pressure and a further lowering of the decrease of pressure within the nozzle.

In principle, a tapering portion of the outlet channel may be provided anywhere along the outlet channel to at least partially achieve the advantages mentioned above. However, in a further preferred embodiment of the spray nozzle according to the invention the outlet channel comprises a tapering portion within which the outlet channel tapers in the flow direction, said tapering portion abutting the exit end of the outlet channel. In other words, the end of the tapering portion forms the exit end of the outlet channel, so that the outlet channel tapers in the flow direction until it reaches the exit end of it. A further reduction of the necessary pump pressure and a further lowering of the decrease of pressure within the nozzle could be achieved hereby. Above this, the described modification improves the spray pattern.

In a further preferred embodiment of the spray nozzle according to the invention the tapering portion does not only abut the exit end of the output channel, the tapering portion rather abuts the entrance end of the outlet channel as well. In other words, the one end of the tapering portion forms the exit end of the outlet channel while the other end of the tapering portion forms the entrance end of the outlet channel, so that the tapering portion runs form the one end of the outlet channel to the other end of the outlet channel. Thus, the whole outlet channel tapers in the flow direction. It has been found out that the spray nozzle could thereby further be improved with regard to the advantageous effects mentioned above.

Due to the production method of the spray nozzle, the edge surrounding the exit end of the outlet channel is rounded, so that it has a radius. In an advantageous embodiment of the spray nozzle according to the invention the edge surrounding the exit end has a radius being smaller than 0.03 mm, preferably smaller than 0.02 mm. It has been found out that the spray nozzle could further be improved with regard to the advantageous effects by limiting the radius of the edge accordingly.

In an advantageous embodiment for a flow rate (for the fluid water is considered) between 0.10 and 0.15 ml/s at 1 bar or approximatively 0.20 and 0.35 ml/s at 6 bar, it results to be advantageous a exit diameter between 0.1 mm and 0.15 mm to achieve an average particle size by volume (DV50), which is equal or lower than 100 micrometer at 1 bar and equal or lower than 60 micrometer at 6 bars, or preferably equal or lower than 95 micrometer at 1 bar and equal or lower than 55 micrometer at 6 bars. For spraying higher particle size at the same pressure, the diameter has to be bigger and proportionally with it, the channel geometry. A bigger geometry of the nozzle, which sprays a higher flow rate, would be even more advantageous in order to achieve smaller particle size at the same pressure and fluid characteristics and would allow the production of even smaller particle size that the ones mentioned above.

In a further advantageous embodiment of the spray nozzle according to the invention the degree of tapering is constant in the flow direction. In this embodiment, it is preferred if at least a tapering portion of the outlet channel or the whole outlet channel has the form of a truncated cone or a truncated pyramid.

In a further advantageous embodiment of the spray nozzle according to the invention the degree of tapering increases in the flow direction. It has been found out that the spray nozzle could further be improved with regard to the advantageous effects mentioned above by increasing the degree of tapering in the flow direction. It especially has a positive effect on the spray pattern and the spray angle without having a negative influence on the pressure drop within the nozzle and the pump pressure necessary to dispense the fluid. Very good results have been achieved by providing an inner face of the outlet channel which is curved in the flow direction, this modification being further preferred in this embodiment. In this connection it is further preferred if at least a tapering portion of the outlet channel or the whole outlet channel more has the form of a spherical layer or a truncated paraboloid of revolution.

In a further and alternative, respectively, embodiment of the spray nozzle according to the invention the degree of tapering decreases in the flow direction. Again, it has been figured out that the spray nozzle could further be improved with regard to the advantageous effects mentioned above by decreasing the degree of tapering in the flow direction. As in the embodiment described hereinbefore, it especially has a positive effect on the spray pattern and the spray angle without a negative influence on the pressure drop within the nozzle and the pump pressure necessary to dispense the fluid. The inner face of the outlet channel is preferably curved in the flow direction. More preferably at least a tapering portion of the outlet channel or the whole outlet channel has the form of a truncated hyperboloid of revolution.

In a further advantageous embodiment of the spray nozzle according to the invention the inner face of the outlet channel includes an angle, said angle preferably varying between 40° and 160°, more preferably between 80° and 120°, most preferably between 90° and 115°, in order to achieve a positive effect on the spray pattern and the minimum pump pressure necessary to dispense the fluid.

In a further preferred embodiment of the spray nozzle according to the invention the feeding channel comprises a first section and a second section following the first section in the flow direction and abutting the swirl chamber. In order to keep up the pressure within the nozzle and to avoid a pressure drop, respectively, the width of the first section preferably decreases in the flow direction. In this embodiment, the width of the second section is preferably constant or decreasing to a lesser extent than the width of the first section in the flow direction. In this embodiment, it is further preferred if the side walls of the first section include an angle, said angle being subdivided into a first angle and a second angle by a centerline of the second section, the maximum difference between the first angle and the second angle being 10°, more preferably 5° or 1°. In the ideal case the first angle corresponds to the second angle. It has been found out that the positive effects mentioned before, especially keeping up the pressure within the nozzle and creating of the swirl within the swirl chamber, could further be enhanced.

In order to further enhance the positive effects of the invention as mentioned before, in a further advantageous embodiment of the spray nozzle according to the invention the length of the second section in the flow direction is equal to or smaller than the width of the second section.

In order to avoid the pressure drop within the nozzle, thereby reducing the minimum pump pressure of the spray nozzle, in a further advantageous embodiment of the spray nozzle according to the invention the height of the first or/and second section is decreasing in the flow direction. In this case it is also preferred if the degree of height reduction is constant, decreasing or increasing in the flow direction. If the degree of height reduction is decreasing or increasing it is further preferred if the bottom or the top of the second section is curved.

In order to facilitate the assembly of the spray nozzle, in a further advantageous embodiment of the invention the spray nozzle is assembled from a first element comprising protrusions for forming the side walls of the feeding channels and grooves between the protrusions and a second element being supported on the protrusions and covering the grooves in order to form the feeding channels. In order to support the second element, the protrusions comprise a support surface. Usually the second element being supported on the protrusions and covering the grooves in order to form the feeding channels is part of the sprayer for which the nozzle is intended to be used.

In order to further facilitate assembly the second element is a separated part which can be jammed or welded into the first element. In this preferred embodiment, to facilitate assembly, the second element can be jammed or welded into the first element and is molded together with the first element in one single part and connected by a flexible connecting piece.

Due to the production method of the spray nozzle, the transition region between the support surface of the protrusions and the surface of the protrusions facing the feeding channel may be rounded, so that it has a radius. In a further preferred embodiment of the spray nozzle according to the invention the ratio of the radius between the support surface of the protrusion and the surface of the protrusion facing the feeding channel to the width of the feeding channel is equal to or less than 1/3, more preferably equal to or less than 1/4, most preferably equal to or less than 1/5, in order to achieve a compact cross-sectional form and to reduce the pressure drop within the nozzle.

In order to facilitate the assembly of the spray nozzle and to ensure a tight support of the second element on the first element, in a further preferred embodiment of the spray nozzle according to the invention one of the first and second element comprises an elastic portion, said elastic portion being elastically deformed by the other element when the elements are assembled. In other words, one of the first and second elements is elastically pressed against the other of the first and second elements when the elements are assembled. This pretension further avoids separation of the first and second element if high pressures occur within the spray nozzle.

In a further advantageous embodiment of the spray nozzle according to the invention the protrusions or/and the section of the first element carrying the protrusions form the elastic portion. The section of the first element carrying the protrusions is preferably a bottom of a cup-like first element being fixed to a surrounding wall of the cup-like first element. In this case, it is further preferred if the bottom is curved or convex towards the second element, before the first and second element are assembled. Said bottom may for example be more elastic than the surrounding wall.

In order to achieve an easily adjustable and flexible spray nozzle with regard to the pump pressure, the volumetric flow, the spray pattern, the spray angle or the like, in a further preferred embodiment of the spray nozzle according to the invention the assembled first and second element are movable relative to each other into different relative positions thereby elastically changing the form, dimensions or/and justification of the feeding channels or/and the swirl chamber. It is further preferred, if the elements are lockable in their different relative positions, so that the change in pump pressure, volumetric flow, spray pattern, spray angle or the like could be kept up without the need to hold both elements in their relative position manually.

In order to further facilitate the production of the spray nozzle and the handling of the same during the assembling, in a further advantageous embodiment of the spray nozzle according to the invention the first element and the second element are connected via a flexible connecting piece. Thus the two elements may be moved relative to each other during the assembling without the risk that one or the other element gets lost. The flexible connecting piece it preferably formed by a strip. It is further preferred, if the connecting piece is integrally formed or moulded with the first and second element or at least a part of the first and second element in order to facilitate the production of the spray nozzle.

In a further advantageous embodiment of the spray nozzle according to the invention an outlet layer with a first hole, a channel layer with a second hole and slots and an inlet layer with holes are provided, said layers being sandwiched such that the first hole forms the outlet channel, the second hole forms the swirl chamber, the slots form the feeding channels and the holes in the inlet layer form inlet holes for feeding the fluid from the fluid chamber into the feeding channels. The layers could for example be integrally formed, e. g. by galvanisation.

In order to allow a quick cleaning and prototyping of the spray nozzle, in a further preferred embodiment of the spray nozzle according to the invention the layers are separable from each other or/and each of the layer is replaceable. Above this, this modification allows to produce a plurality of combinations of layers out of a few prefabricated layers without the need to provide the same number of single layers. The separable or/and replaceable layers could for example be provided in the form of thin discs.

In order to provide a spray nozzle being flexible with regard to the pump pressure, the volumetric flow, the spray pattern, the spray angle or the like, in a further preferred embodiment of the spray nozzle according to the invention there is provided an overlapping area between the inlet holes and the feeding channels, in order to feed the fluid through the inlet holes into the feeding channels. The size of the overlapping area or/and the distance between the overlapping area and the swirl chamber is preferably adjustable. By changing the size of the overlapping area or/and the distance between the overlapping area and the swirl chamber, the spray pattern or the like could be easily changed. In order to facilitate the handling of the spray nozzle, the inlet layer and the channel layer are more preferably moveable, most preferably rotatable, relative to each other in order to adjust the size of the overlapping area or/and the distance between the overlapping area and the swirl chamber. As alternative in order to further facilitate the handling of the spray nozzle, the inlet layer and the channel layer are more preferably moveable, by snapping them, rotating them on their axis to adjust the size of the overlapping area or shifting them by lateral movement to exchange a layer.

The sprayer according to the invention, e.g. an easy sprayer, a continuous sprayer, a squeeze sprayer, a sprayer with a pressurized fluid storage container or a low energy electrical sprayer, comprises an embodiment of the spray nozzle according to the invention. The advantages mentioned in connection with the spray nozzle apply mutatis mutandis.

In a preferred embodiment of the sprayer according to the invention the sprayer is a hand operated sprayer, the sprayer preferably comprising a fluid storage container being manually squeezable or another manually actuatable pumping device. Alternatively, the sprayer is an electrically driven sprayer. In both cases, due to the advantages of the spray nozzle there is no need to apply a high pump force, so that actuation is easier and less energy consuming.

### BRIEF DESCRIPTION OF THE DRAWINGS

Preferred embodiments of the present invention will now be described, by way of example only, with reference to the drawings in which
Fig. 1 shows a cross-sectional side-view of a first embodiment of the spray nozzle;
Fig. 2 shows a cross-sectional view along line A-A in Fig. 1;
Fig. 3 shows a cross-sectional view along line B-B in Fig. 2;
Fig. 4 shows the enlarged section A of Fig. 1;
Fig. 5 shows the enlarged section A of Fig. 1 with a first modification;
Fig. 6 shows the enlarged section A of Fig. 1 with a second modification;
Fig. 7 shows a schematic view of a second embodiment of the spray nozzle; and
Fig. 8 shows a schematic view of a third embodiment of the spray nozzle.

### DETAILED DESCRIPTION OF THE INVENTION

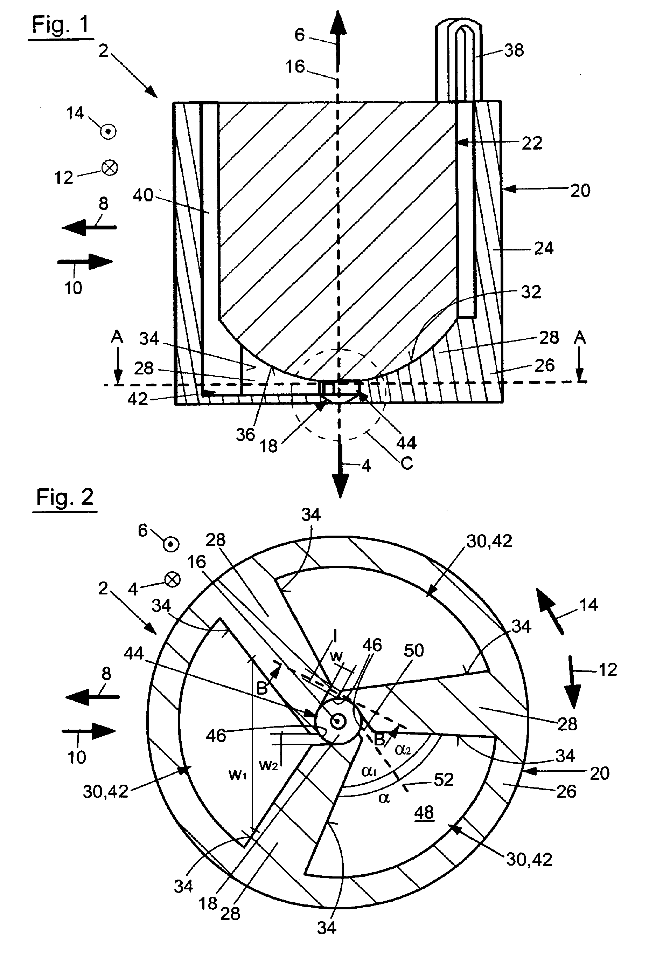

Fig. 1 to 4 show views of a first embodiment of the spray nozzle 2 for dispensing a fluid. In the figures, the opposing longitudinal directions 4, 6, the opposing radial directions 8, 10 and the opposing circumferential directions 12, 14 of the spray nozzle are indicated by corresponding arrows. The longitudinal axis 16 of the spray nozzle 2 extends in the longitudinal directions 4, 6, said longitudinal axis 16 further forming the centre axis of the outlet channel 18.

The spray nozzle 2 is assembled from a first element 20 and a second element 22. The first element 20 has a cup-like structure with a first section 24 extending in the circumferential directions 12, 14 and forming a surrounding wall and a second section 26 forming the bottom of the cup-like structure. The second section further comprises protrusions 28, said rib-like protrusions 28 extending in the longitudinal direction 6 and in the radial directions 8, 10. As can be best seen in Fig. 2, there are provided grooves 30 in the circumferential directions 12, 14 between the protrusions 28, said grooves being provided to form the feeding channels as will be described later. The protrusions 28 comprise an upper surface serving as a support surface 32 for supporting the second element 22, said support surface 32 facing the second element 22. Further, the protrusions 28 comprise side surfaces 34 facing the grooves 30 and feeding channels, respectively.

The second element 22 basically has a cylindrical form with a front face 36, said front face 36 bulging out in the longitudinal direction 4. In this embodiment, the front face 36 has a form of a spherical cap. The second element 22 is inserted into the cup-like first element 20, so that the front face 36 is supported on the support surfaces 32 of the protrusions 28. In this connection it should be mentioned, that the second element 22 may also be formed by a ball, which is pressed or clipped into the cup-like first element 20. Independent of the chosen form of the second element 22, it is preferred if the second element 22 could be snapped or clicked into its place within the first element 20, even if corresponding notches, snaps or the like for providing a form-fit or/and a force-fit are not shown in the figures.

The first element 20 and the second element 22 are connected via a flexible connecting piece 38, which - in this case - is formed by a strip. The connecting piece 28 is integrally formed or moulded with the second element 22 and at least the first section 24 of the first element 20. Even the second section 26 of the first element 20 may be integrally formed or moulded with the first section 24 of the first element 20 and consist of the same material. However, in this case the second section 26 has been subsequently fastened to the first section 24 since the second section 26 is made of a different material, as will be described hereinafter. Irrespective of the second section 26 being integrally formed with the first section 24 or not, the first element 20 comprises an elastic portion.

As already indicated above, the first element 20 is at least partially made of an elastic material being more elastic than the material of the second element 22. In this case, the second section 26 of the first element 20 with its protrusions 28 and its bottom section carrying said protrusions 28 is made of the elastic material, said elastic material being more elastic than the material of the second element 22 and more elastic than the material of the first section 24 of the first element 20. Thus, the afore-mentioned elastic portion of the first element 20 is essentially formed of the protrusions 28 and its bottom section carrying said protrusions 28. The elastic portion of the first element 20 is elastically deformed by the second element 22 when the elements 20, 22 are assembled. Even if the pre-assembled state is not shown, it is preferred if the bottom section carrying said protrusions 28 is curved or convex towards the second element 22 and in the longitudinal direction 6, respectively, before the first and second element 20, 22 are assembled.

The spray nozzle 2 is assembled by inserting the second element 22 into the cup-like first element 20 in the longitudinal direction 4 as shown in Fig. 1, thereby creating a fluid chamber 40, feeding channels 42 and a swirl chamber 44, while the outlet channel 18 is already provided in the second section 26 of the first element 20. The fluid chamber 40 is positioned in the radial directions 8, 10 between the first section 24 of the first element 20 and the second element 22, so that the fluid chamber 40 is formed as a ring chamber. The fluid chamber 40 receives the fluid to be dispensed from a fluid storage chamber or container, which is not shown in the drawings. In the longitudinal direction 4 the fluid chamber 40 abuts the radial outer ends of the feeding channels 42, so that there is a fluid connection between the fluid chamber 40 and the feeding channels 42.

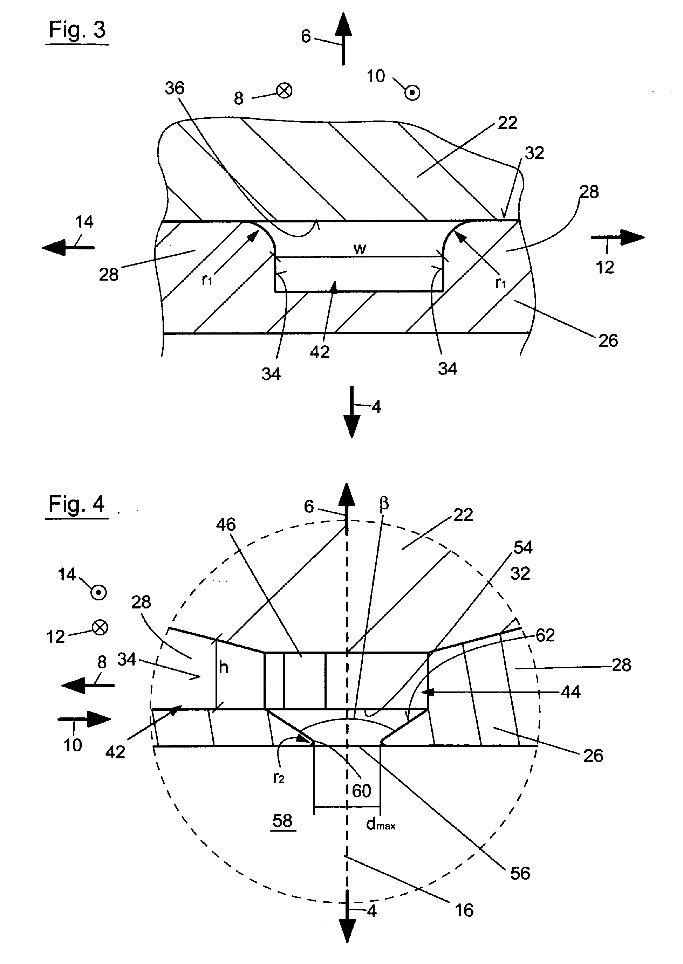

As can especially be seen in Fig. 2, the feeding channels 42 are extending radially inward to an exit end 46 of the feeding channels 42, where the feeding channels 42 abut the swirl chamber 44, so that the fluid may be fed from the fluid chamber 40 via the feeding channels 42 into the swirl chamber 44. As shown in Fig. 3, the feeding channels 42 are limited in the circumferential directions 12, 14 by the side surfaces 34 of the protrusions 28, in the longitudinal direction 6 by the front face 36 of the second element 22, said second element 22 covering the grooves 30 to form the feeding channels 42, and in the longitudinal direction 4 by the bottom of the second section 26 carrying the protrusions 28.

The feeding channels 42 comprise a first section 48 abutting the fluid chamber 40 and a second section 50 following the first section 48 in the flow direction and radial direction 10, respectively. The second section 50 abuts the swirl chamber 44 with the exit end 46. As shown in Fig. 3, the width w1 of the first section 48 decreases in the flow direction and the radial direction 10, respectively. In contrast to this, the width w2 of the second section 50 is constant or decreases to a lesser extent than the first section 48 in the flow direction and radial direction 10, respectively.

The protrusions 28, which form the side walls of the first sections 48, include an angle α. In Fig. 3, there is further indicated a centerline 52 of the second section 50 extending in the radial directions 8, 10. Said centerline 52 subdivides the angle α into a first angle α1 and a second angle α2. The maximum difference between the first angle α1 and the second angle α2 is 10°, more preferably 5° or 1°. Due to the bulged out front face 36 of the second element 22, at least the height h of the first section 48 of the feeding channels 42 decreases in the flow direction and the radial direction 10, respectively. Further, the length 1 of the second section 50 in the flow direction and the radial direction 10, respectively, is equal to or smaller than the width w2 of the second section 50.

As shown in Fig. 3, in the transition region between the support surfaces 32 and the side surfaces 34 the protrusions 28 comprise a radius r1. In order to have a compact cross-sectional form, the ratio of the radius r1 to the width w, e. g. w1 or w2, of the feeding channel 42 is equal to or less than 1/3, more preferably equal to or less than 1/4, most preferably equal to or less than 1/5.

Even if the first element 20 and the second element 22 are assembled, they are still movable relative to each other into different relative positions. In the shown embodiment, the elements 20 and 22 may be moved in the longitudinal direction 4, 6 relative to each other. By this relative movement the form, dimensions or/and justification of the feeding channels 42 or/and the swirl chamber 44 is changed by elastically deforming the protrusions 28 or/and the bottom of second section 26 of the first element 20, i.e. by elastically deforming the elastic portion of the first element 20. In other words, it is easy to change the behaviour of the spray nozzle 2. Further, there are provided means (not shown) for locking the elements 20, 22 in their different relative positions.

With reference to Fig. 4, the afore-mentioned outlet channel 18 in the second section 26 of the first element 20 comprises an entrance end 54 facing the swirl chamber 44 in the longitudinal direction 6 and an exit end 56 for discharging the fluid to the environment 58 of the spray nozzle 2 and the sprayer, respectively, in the longitudinal direction 4. The outlet channel 18 tapers steadily in the flow direction and the longitudinal direction 4, respectively. Thus, the outlet channel 18 comprises at least one tapering portion. In the shown embodiment, the tapering portion abuts the exit end 56 as well as the entrance end 54 of the outlet channel 18, so that the whole outlet channel tapers in the flow direction. The edge 60 surrounding the exit end 56 has a radius r2. The radius r2 is smaller than 0.03 mm, preferably smaller than 0.02 mm. Further, the exit end 56 has a maximum diameter between 0.1 mm and 0.15 mm and more preferably a corresponding maximum cross-sectional area to achieve a average particle size by volume (DV50), which is equal or lower than 100 micrometer at 1 bar and equal or lower than 60 micrometer at 6 bars, or preferably equal or lower than 95 micrometer at 1 bar and equal or lower than 55 micrometer at 6 bars. Between a flow rate (for the fluid water is considered) between 0.10 and 0.15 ml/s at 1 bar or approximately 0.20 and 0.35 ml/s at 6 bar. A bigger geometry or the nozzle, which sprays a higher flow rate, would be even more advantageous in order to achieve smaller particle size at the same pressure and fluid characteristics and would allow the production of even smaller particle size that the ones mentioned above. Above this, the outlet channel 18 has an inner face 62 surrounding the outlet channel 18 and limiting the same in the radial direction 8. The inner face 62 of the outlet channel 18 includes an angle β, said angle β preferably varying between 40° and 160°, preferably between 80° and 120°, more preferably between 90° and 115°.

As shown in Fig. 4, the degree of tapering of the outlet channel 18 is constant in the flow direction and the longitudinal direction 4, respectively. In the shown embodiment this is achieved by at least a tapering portion of the outlet channel 18 or the whole outlet channel 18 having the form of a truncated cone or a truncated pyramid. It has further been found out, that the pressure drop in the spray nozzle 2 could be reduced and a further reduction of the minimum pump pressure for dispensing the fluid could be achieved by adjusting the ratio of the sum of the cross-sectional areas of the feeding channels 42 at their exit end 46 to the cross-sectional area of the exit end 56 of the output channel 18.

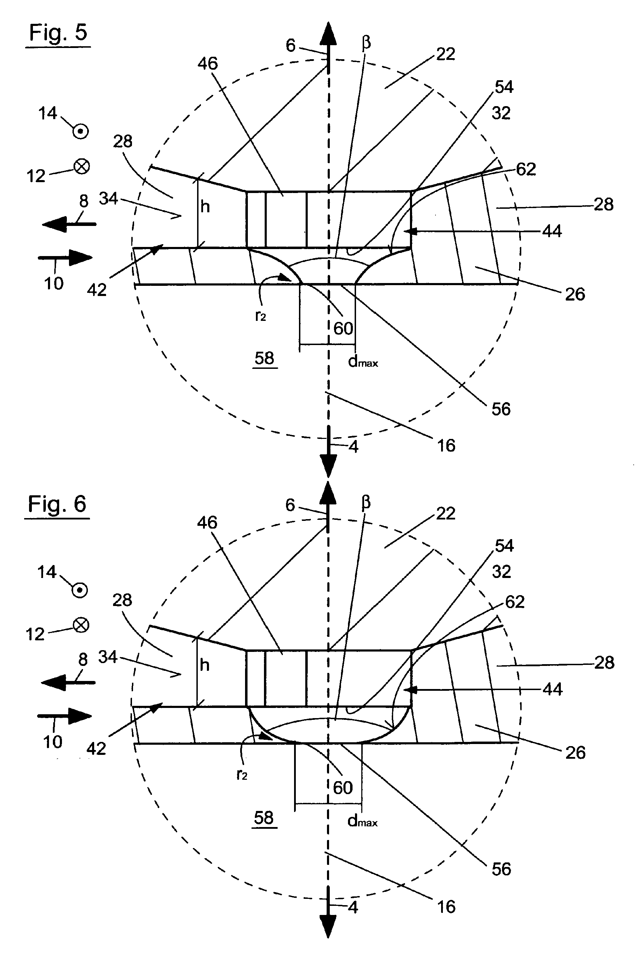

Fig. 5 shows the enlarged section A of Fig. 1 with a first modification. In the following only the differences will be described, the same reference signs will be used for similar or the same components and the above description of the first embodiment applies accordingly in this regard.

In contrast to the outlet channel 18 described with reference to Fig. 1 to 4, the degree of tapering of outlet channel 18 according to Fig. 5 decreases in the flow direction and the longitudinal direction 4, respectively. This is achieved by providing an inner face 62 of the outlet channel 18 being curved in the flow direction and the longitudinal direction 4, respectively. In the embodiment according to Fig. 5, at least the tapering portion of the outlet channel 18 or the whole outlet channel 18 has the form of a truncated hyperboloid of revolution.

Fig. 6 shows the enlarged section A of Fig. 1 with a second modification. In the following only the differences will be described, the same reference signs will be used for similar or the same components and the above description of the first embodiment applies accordingly in this regard.

In contrast to the outlet channel 18 described with reference to Fig. 1 to 4, the degree of tapering of outlet channel 18 according to Fig. 6 increases in the flow direction and the longitudinal direction 4, respectively. This is achieved by providing an inner face 62 of the outlet channel 18 being curved in the flow direction and the longitudinal direction 4, respectively. In the embodiment according to Fig. 6, at least a tapering portion of the outlet channel or the whole outlet channel has the form of a spherical layer or a truncated paraboloid of revolution.

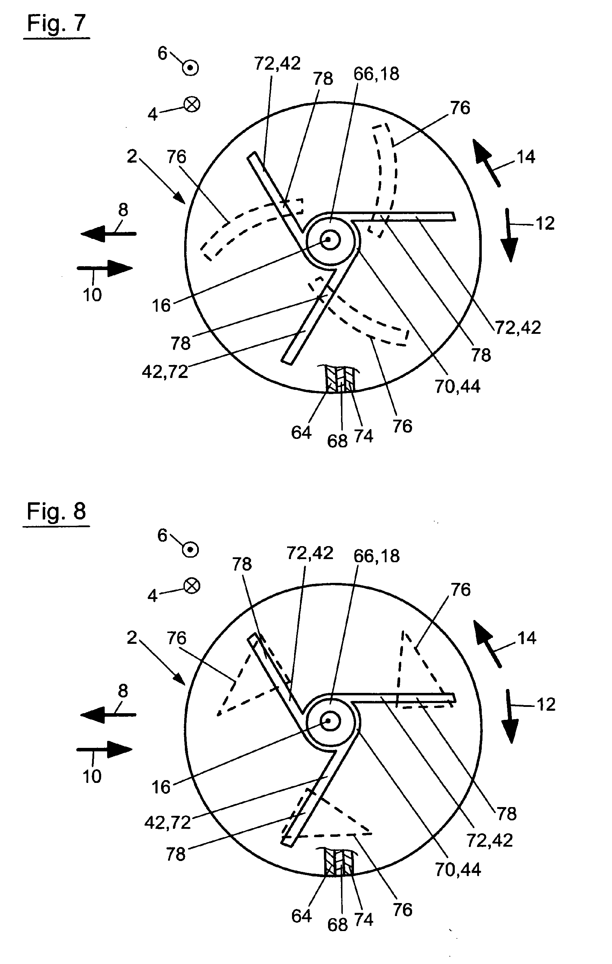

Fig. 7 shows a second embodiment of the spray nozzle according to the invention. Since the second embodiment at least partially corresponds to the first embodiment according to Fig. 1 to 6, in the following only the differences will be described, the same reference signs will be used for similar or the same components and the above description of the first embodiment applies accordingly in this regard.

The spray nozzle 2 according to Fig. 7 comprises at least three layers, i.e. an outlet layer 64 with a first hole 66, a channel layer 68 with a second hole 70 and slots 72 and an inlet layer 74 with slot-like holes 76, said layers 64, 68 and 74 being sandwiched, while the inlet layer 74 is shown in a transparent manner in Fig. 7 to increase the intelligibility of the drawing. Being sandwiched this way, the first hole 66 forms the outlet channel 18, the second hole 70 forms the swirl chamber 44, the slots 72 form the feeding channels 42 and the holes 76 in the inlet layer form inlet holes for feeding the fluid from the fluid chamber 40 into the feeding channels 42. In the shown embodiment, the layers 64, 68 and 74 are separable from each other and each of the layers 64, 68 and 74 could be replaced, so that the layers 64, 68 and 74 could also be regarded as separate discs with corresponding slots and holes.

As shown in Fig. 7, there is provided an overlapping area 78 between the inlet holes 76 and the j feeding channels 42 when viewed in the longitudinal direction 4. The inlet layer 74 and the channel layer 68 are moveable - in this case rotatable around the longitudinal axis 16 - relative to each other, while the inlet holes 76 and the feeding channels 42 are formed such that, the distance between the overlapping area 78 and the swirl chamber 44 could be reduced by rotating the inlet layer 74 relative to the channel layer 68 in the circumferential direction 14 and could be enlarged by rotating the inlet layer 74 relative to the channel layer 68 in the circumferential direction 12. Thus, the distance between the overlapping area 78 and the swirl chamber 44 is adjustable.

Fig. 8 shows a third embodiment of the spray nozzle 2 according to the invention. Since the third embodiment at least partially corresponds to the second embodiment according to Fig. 7, in the following only the differences will be described, the same reference signs will be used for similar or the same components and the above description of the first and second embodiment applies accordingly in this regard.

In contrast to the second embodiment, the inlet holes 76 and the feeding channels 42 of the third embodiment are formed such that, the size of the overlapping area 78 could be reduced by rotating the inlet layer 74 relative to the channel layer 68 in the circumferential direction 12 and could be enlarged by rotating the inlet layer 74 relative to the channel layer 68 in the circumferential direction 14. Thus, the size of the overlapping area 78 is adjustable.

It should be mentioned that the principles of the second and third embodiment could also be advantageously combined in a single spray nozzle 2, so that the size of the overlapping area 78 as well as the distance between the overlapping area 78 and the swirl chamber 44 could be adjusted by a relative movement between the inlet layer 74 and the channel layer 68.

According to the invention, the spray nozzle 2 should be used in a sprayer, said sprayer preferably being a hand operated sprayer, the sprayer more preferably comprising a fluid container being manually squeezable, a sprayer with a pressurized fluid storage container or a manually actuatable pumping device, or in an electrically driven sprayer.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. Spray nozzle (2) for dispensing a fluid comprising a fluid chamber (40), preferably a ring chamber, for receiving the fluid, at least one feeding channel (42) for feeding the fluid from the fluid chamber (40) radially inward into a swirl chamber (44) and an outlet channel (18) with an entrance end (54) facing the swirl chamber (44) and an exit end (56) for discharging the fluid to the environment (58) of the spray nozzle (2),
**characterized in that**
the outlet channel (18) tapers in the flow direction of the fluid.

2. Spray nozzle (2) according to claim 1, **characterized in that**
the outlet channel (18) tapers steadily or/and a tapering portion of the outlet channel (18) abuts the exit end (56), the tapering portion preferably abutting the entrance end (54) as well.

3. Spray nozzle (2) according to one of claims 1 or 2, **characterized in that** an edge (60) surrounding the exit end (56) has a radius (r2) being smaller than 0.03 mm, preferably smaller than 0.02 mm, or/and the exit end (56) has a maximum diameter (dmax) between 0.1 and 0.2 mm, more preferably between 0.12 and 0.18 mm.

4. Spray nozzle (2) according to one of claims 1 to 3, **characterized in that** the degree of tapering is either constant in the flow direction, preferably wherein at least a tapering portion of the outlet channel (18) or the whole outlet channel (18) having the form of a truncated cone or a truncated pyramid,
or the degree of tapering increases in the flow direction, preferably wherein the inner face (62)of the outlet channel (18) being curved in the flow direction, more preferably wherein at least a tapering portion of the outlet channel (18) or the whole outlet channel (18) having the form of a spherical layer or a truncated paraboloid of revolution,
or the degree of tapering decreases in the flow direction, preferably wherein the inner face (62) of the outlet channel (18) being curved in the flow direction, more preferably wherein at least a tapering portion of the outlet channel (18) or the whole outlet channel (18) having the form of a truncated hyperboloid of revolution.

5. Spray nozzle (2) according to one of claims 1 to 4, **characterized in that** the inner face (62) of the outlet channel (18) includes an angle (β), said angle (β) preferably varying between 40° and 160°, more preferably between 80° and 120°, most preferably between 90° and 115°.

6. Spray nozzle (2) according to one of the preceding claims, **characterized in that** the feeding channel (42) comprises a first section (48) and a second section (50) following the first section (48) in the flow direction and abutting the swirl chamber (44), the width (w1) of the first section (48) preferably decreasing in the flow direction and the width (w2) of the second section (50) preferably being constant or decreasing to a lesser extent in the flow direction, the side walls of the first section (48) more preferably including an angle (α), said angle (α) being subdivided into a first angle (α1) and a second angle (α2) by a centerline (52) of the second section (50), the maximum difference between the first angle (α1) and the second angle (α2) being 10°, most preferably 5° or 1°.

7. Spray nozzle (2) according to claim 6, **characterized in that**
the length (1) of the second section (50) in the flow direction is equal to or smaller than the width (w2) of the second section (50) or/and the height (h) of the first or/and second section (48; 50) is decreasing in the flow direction.

8. Spray nozzle (2) according to one of the preceding claims, **characterized in that** the spray nozzle (2) is assembled from a first element (20) comprising protrusions (28) for forming the side walls of the feeding channels (42) and grooves (30) between the protrusions (28) and a second element (22) being supported on the protrusions (28) and covering the grooves (30) in order to form the feeding channels (42), the protrusions (28) comprising a support surface (32) for supporting the second element (22), the ratio of the radius (r1) between the support surface (32) and the surface (34) of the protrusions (28) facing the feeding channel (42) to the width (w) of the feeding channel (42) preferably being equal to or less than 1/3, more preferably equal to or less than 1/4, most preferably equal to or less than 1/5.

9. Spray nozzle according to any of the preceding claims, **characterized in that** the bottom is conical in longitudinal direction (6) forming with the second part (22) a contact area which is defined by the penetration of the second part during the assembly, which generate pretension between the two parts due slightly bending the bottom of the nozzle in longitudinal direction (4).

10. Spray nozzle (2) according to claim 9, **characterized in that**
one of the first and second element (20, 22) comprises an elastic portion, the elastic portion being elastically deformed by the other element (22, 20) when the elements (20, 22) are assembled, the protrusions (28) or/and the section of the first element (20) carrying the protrusions (28) preferably forming the elastic portion.

11. Spray nozzle (2) according to claim 10, **characterized in that**
the assembled elements (20, 22) are movable relative to each other into different relative positions thereby elastically changing the form, dimensions or/and justification of the feeding channels (42) or/and the swirl chamber (44), the elements (20, 22) preferably being lockable in the different relative positions.

12. Spray nozzle (2) according to one of claims 9 to 11, **characterized in that** the first element (20) and the second element (22) are connected via a flexible connecting piece (38), preferably a strip, the connecting piece (38) more preferably being integrally formed or moulded with the first and second element (20, 22).

13. Spray nozzle (2) according to one of claims 1 to 8, **characterized in that** an outlet layer (64) with a first hole (66), a channel layer (68) with a second hole (70) and slots (72) and an inlet layer (74) with holes (76) are provided, said layers (64, 68, 74) being sandwiched such that the first hole (66) forms the outlet channel (18), the second hole (70) forms the swirl chamber (44), the slots (72) form the feeding channels (42) and the holes (76) in the inlet layer (74) form inlet holes for feeding the fluid from the fluid chamber (40) into the feeding channels (42), the layers (64, 68, 74) preferably being separable from each other or/and each of the layers (64, 68, 74) preferably being replaceable.

14. Spray nozzle (2) according to claim 13, **characterized in that**
there is provided an overlapping area (78) between the inlet holes and the feeding channels (42), the size of the overlapping area (78) or/and the distance between the overlapping area (78) and the swirl chamber (44) preferably being adjustable, the inlet layer (74) and the channel layer (68) more preferably being moveable, most preferably rotatable, relative to each other in order to adjust the size of the overlapping area (78) or/and the distance between the overlapping area (78) and the swirl chamber (44).

15. Sprayer comprising a spray nozzle (2) according to one of the preceding claims, said sprayer preferably being a hand operated sprayer, a sprayer with a pressurized fluid storage container the sprayer more preferably comprising a fluid container being manually squeezable or a manually actuatable pumping device, or an electrically driven sprayer.
